# EUROPEAN PATENT APPLICATION

(11) **EP 0 816 309 A1**
(43) Date of publication of application: **07.01.1998**
(21) Application number: 97304048.8
(22) Date of filing: 11.06.1997
(51) Int. Cl.: C07B 61/00, C07C 273/18, C07C 335/04, B01L 3/00, B01J 19/00, C07D 307/14, C07D 211/58, C07D 209/16

(54) **Scavenger assisted combinatorial process for preparing libraries of urea and thiourea compounds**

(30) Priority: 14.06.1996 US 19791 P
(71) Applicant: ELI LILLY AND COMPANY, Indianapolis, Indiana 46285 (US)
(72) Inventor: Kaldor, Stephen Warren, Indianapolis, Indiana 46254 (US); Fritz, James Erwin, McCordsville, Indiana 46055 (US)
(74) Representative: Hudson, Christopher Mark

(57) **Abstract**

This invention relates to a novel solution phase process for the preparation of urea or thiourea combinatorial libraries. These libraries have utility for drug discovery and are used to form wellplate components of novel assay kits.

## Description

This invention relates to a solution phase synthesis of combinatorial libraries of ureas and thioureas. These libraries are useful for discovery of lead compounds for drug development.

Research and development expenses account for the largest outlay of capital in the drug industry. Synthesis of compounds is an expensive and time consuming phase of research and development. Historically, research chemists individually synthesized and analyzed hundreds of high purity compounds for biological screening to develop pharmaceutical leads. Although past methods brought new drugs to market, the limitations of individual synthesis and insistence on compound characterization considerably slowed the discovery process.

The need for more rapid and less expensive drug discovery methodology is increasingly important in today's competitive drug industry.

More recently, modern drug discovery has used the methods of combinatorial chemistry to generate large numbers (viz., about 10² to 10⁶) of compounds generically referred to as "libraries." An important objective of combinatorial chemistry is to generate lead compounds for pharmaceutical research.

Theoretically, the total number of compounds which may be produced for a given library is limited only by the number of reagents available to form substituents on the variable positions on the library's molecular scaffold. The combinatorial process lends itself to automation, both in the generation of compounds and their biological screening.

Combinatorial chemistry may be performed in a manner where libraries of compounds are generated as mixtures with complete identification of individual compounds postponed until after positive screening results are obtained. However, a preferred form of combinatorial chemistry is "parallel array synthesis" where individual reaction products (most often individual compounds) are synthesized together, but are retained in separate vessels. For example, the library compounds are held in the individual wells of 96 well microtiter plates. Use of standardized microtiter plates or equivalent apparatus is advantageous because such an apparatus is readily manipulated by programmed robotic machinery.

Conventionally, combinatorial chemistry is conducted on a solid phase support, normally a polymer. The library scaffold is cleavably tethered to the solid support by a chemical linker. Reactions are carried out to modify the scaffold while tethered to the solid support. In a final step, the product is cleaved and released from the solid support. A general procedure for conventional solid phase synthesis is illustrated by the following scheme where the shaded circle symbol is, for example, a high molecular weight polymer:

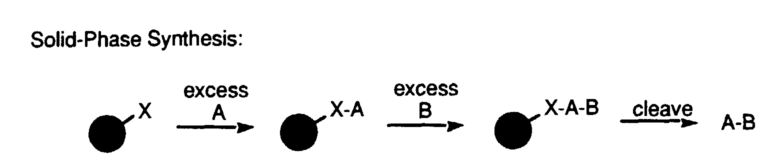

Variations in reagents (e.g., "A", "B", in the general scheme, supra.) produce the desired structural diversity. Separation of solid phase product and unreacted soluble reactant is done by simple separation techniques such as filtration, decantation, or washing. These separation solid phase synthesis techniques have general applicability to a wide variety of combinatorial reactants and lend themselves to large scale simultaneous/automated library creation.

The rate determining step in small molecule synthesis is typically not actual construction of the desired new chemical entities. Rather, the difficulty of synthesis is frequently caused by the task of isolating reaction product from unreacted starting materials, by-products or other impurities.

Unfortunately, it is not always practicable to tether a desired combinatorial scaffold to a solid support. A significant number of combinatorial reaction schemes are desirably done in solution phase. Moreover, not all desired solution phase reactions are driven to completion using near stoichiometric ratios of reactants. Frequently, one reagent is added in considerable excess to drive a solution phase reaction to completion. The result is a reaction medium with soluble product and soluble unreacted co-reactant. Consequently, traditional organic synthetic methods often require complex purification strategies which limit their use, particularly in combinatorial syntheses.

Polymeric reagents have been known and used for general chemical synthesis in various roles; such as follows:
a) The article, *Cyanoborohydride supported anion exchange resin as a selective reducing agent* by Hutchins, Robert O.: Natale, Nicholas R. and Taffer, Ira M.; J.C.S. Chem Comm., pg. 1088-9, 1978 describes various reactions including reductive amination using cyanoborohydride anion on ion-exchange resin. The spent resin reagent is removed by simple filtration and washing.
b) The article, *Synthesis and Reactivity of Polymer-Supported Reducing Agents with Chemically Modified Surfaces* by Menger, Fredric M., Hiraku, Shinozaki, and Lee, Hsueh-Chi; J. Org. Chem 1980, 45, 2724-2725 describes borohydride and cyanoborohydride functional anion exchange resins for carbonyl group reduction. Aldehydes and ketones are reduced to form alcohols by use of polymeric reagents.
c) US Patent No. 3,873,621 describes reductive amination reactions carried out using alkali-metal and quaternary ammonium cyanoborohydrides.
d) The article, *The reduction of α,β- unsaturated nitroalkenes to nitroalkanes with borohydride supported on an ion exchange resin,* by Goudgaon, Naganna, M.; Wadgaonkar, Prakash P.; and Kabalka, George W.; Synthetic Communications, 19(5&6), 805-811 (1989) describes the use of polymer supported borohydride reagent used to reduce nitroalkenes to nitroalkanes.
e) The article, *Borohydride reducing agent derived from anion exchange resin: Selective reduction of α,β-carbonyl compound by* Sande, A.R. et. al., Tetrahedron Letters, Vol. 25, No. 32, pp 3501-3504 1984 describes the use of borohydride exchange resin for the reduction of cyclic and acyclic ketones to alcohols with the attendant advantage of simple separation by filtration to give product free of boron moiety.
f) The article, *A reductive amination/lactamization procedure using borohydride reagents* by Abdel-Magid, Ahmed F.; Harris, Brice D. and Maryanoff, Cynthia A.; Synlett, pgs. 81-3, January 1994 describes reductive amination of carbonyl compounds using sodium triacetoxyborohydride.
g) The article, *New Probes for the Study of Acylation Reactions,* by J. Rebek, D. Brown, and S. Zimmerman (Contribution No. 3481), Journal of the American Chemical Society, 97:15, p.4408, July 23, 1975, JACS 97:15 July 23, 1975 describes the use of polymer bound isocyanate to activate carboxylic acid.
h) The article, *Chemical Modification of Polymers. Borohydride Reducing Agents Derived from Anion Exchange Resins,* by H. W. Bibson and F.C. Bailey, J.C.S. Chem. Comm., p. 815, 1977 describes the preparation of an insoluble polymer bound reducing agent by reacting anion exchange resins of the quaternary ammonium type with aqueous NaBH4.
i) The article, *Solid Phase Synthesis of Oligosaccharides. I. Preparation of the Solid Support. poly(p-(1-propen-3-ol-1-yl) styrene,* by J. M. Frechet and C. Schuerch, Journal of the American Chemical Society, 93:2, p. 492-496 describes the preparation of -CHO functional polymers by contacting chloromethylated resin with methyl sulfoxide and sodium bicarbonate.
j) U.S. Patent No. 3,576,870 describes the purification of dimethylacetamide by removing acetic anhydride with a basic ion exchange resin containing primary or secondary amino groups.
k) The article, *Use of Polymeric Nucleophiles for the Selective Binding and Removal of* α*-Methylene-*γ*-butyrolactone Allergens from Complex Mixtures,* by A. Cheminat and C. Benezra, Tetrahedron Letters, Vol. 21, p. 617-619 (1980) describes an amine functional polymer used as a nucleophile for removal of an α,β-unsaturated lactone electrophile.
l) The article, *Polymeric De-blocking Agents for the Fluoren-9-ylmethoxycarbonyl (FMOC) Amino-protecting Group,* by L.S. Carpino and J.R. Williams, J.C.S. Chem. Comm., p.450-451, (1978) describes the use of a resin bound piperazine to remove FMOC with subsequent scavenging of the dibenzofulvene contaminant.
m) The article, *Piperazino-Functionalized Silica Gel as a Deblocking-Scavenging Agent for the 9-Fluorenylmethyloxycarbonyl Amino-Protecting Group,* by L.A. Carpino, E.M.E. Mansour, and J. Knapczyk, J. Org. Chem., 48, p.666-669 (1983) describes a silica bound piperazine to remove FMOC with subsequent scavenging of the dibenzofulvene contaminant.
n) The article, Preparation of High Capacity Aminomethyl-Polystyrene Resin, by C. C. Zikos and N.G. Ferderigos, Tetrahedron Letters, Vol. 36, No. 21, p. 3741-3744, 1995, describes the preparation of an amine functional resin.
o) U.S. Patent No. 5,244,582 relates to reactive groups immobilized on inorganic substrates such as glass. Such immobilized groups can be used to remove nitrosating agents in liquids, etc.

There remains a need to develop solution phase combinatorial processes for making libraries.

Combinatorial chemistry may be used at two distinct phases of drug development. In the discovery phase highly diverse libraries are created to find lead compounds. In a second optimization phase, strong lead compounds are much more narrowly modified to find optimal molecular configurations. The method of this invention has applicability for making both diverse libraries of urea and thiourea compounds useful for finding new lead compounds, and directed libraries of ureas and thioureas useful for optimizing a particular desired biological activity.

FIG. 1 is a top view of a wellplate apparatus.

FIG. 2 is a side view of a wellplate apparatus.

### Detailed Description of the Invention

### I. Definitions:

The following terms have the meaning defined below when used in this specification of the invention:

"Assay kit" means an assemblage of two cooperative elements, namely, (i) a wellplate apparatus, and (ii) biological assay materials.

"Biological assay materials" are materials necessary to conduct a biological evaluation of the efficacy of any library compound in a screen relevant to a selected disease state.

"Directed Library" is a collection of compounds created by a combinatorial chemistry process for the purpose of optimization of the activity of a lead compound, wherein each library compound has a common scaffold, and the library, considered in its entirety, is a collection of closely related homologues or analogues to the lead compound (compare to "Diverse library").

"Diverse library" means a library where the substituents on the combinatorial library scaffold are highly variable in constituent atoms, molecular weight, and structure and the library, considered in its entirety, is not a collection of closely related homologues or analogues (compare to "Directed library").

"Lead compound" means a compound in a selected combinatorial library for which the Assay kit has revealed significant activity relevant to a selected disease state.

"Library" is a collection of compounds created by a combinatorial chemical process, said compounds having a common scaffold with one or more variable substituents.

"Library compound" means an individual reaction product (usually a single compound) in a library produced by the method of the invention, either the urea or thiourea libraries.

"Parallel array synthesis" means a method of conducting combinatorial chemical synthesis of libraries wherein the individual combinatorial library reaction products are separately prepared and stored without prior or subsequent intentional mixing.

"Reaction zone" means the individual vessel location where the combinatorial chemical library compound preparation process of the invention is carried out and individual library compounds synthesized. Suitable reaction zones are the individual wells of a wellplate apparatus.

"Scaffold" means the invariant region (viz., core) of the compounds which are members of a library (viz., urea or thiourea).

"Simultaneous synthesis" means making of library of compounds within one production cycle of a combinatorial method (not making all library compounds at the same instant in time).

"Solid-supported scavenger" means a reaction medium insoluble solid substance containing chemical functionality reactive with the soluble impurity (viz., usually excess reactant) desired to be removed from the reaction medium in the presence of soluble product.

"Substituents" are chemical radicals which are bonded to the scaffold through the combinatorial synthesis process. The different functional groups account for the diversity of molecules throughout the library and are selected to impart diversity of biological activity to the scaffold in the case of diverse libraries, and optimization of a particular biological activity in the case of directed libraries.

"Reagent" means a reactant, any chemical compound used in the combinatorial synthesis to place substituents on the scaffold of a library.

"Wellplate apparatus" means a structure capable of holding a plurality of library compounds in dimensionally fixed and defined positions.

"Non-interfering substituent", refers to an organic (non-hydrogen) radical suitable for substitution as R₁, R₂, R₃ or R₄ in the reactants used in the process of making a combinatorial urea or thiourea library. Non-interfering substituents are those that do not significantly impede the solution phase processes of the invention or interfere with the use of a solid phase scavenger in said processes. Suitable non-interfering radicals include, but are not limited to, C₁-C₁₀ alkyl, C₂-C₁₀ alkenyl, C₂-C₁₀ alkynyl, C₁-C₁₀ alkoxy, C₇-C₁₂ aralkyl, C₇-C₁₂ alkaryl, C₃-C₁₀ cycloalkyl, C₃-C₁₀ cycloalkenyl, phenyl, substituted phenyl, toluyl, xylenyl, biphenyl, C₂-C₁₂ alkoxyalkyl, C₁-C₆ alkylsulfinyl, C₁-C₁₀ alkylsulfonyl, -(CH₂)ₘ-O-(C₁-C₁₀ alkyl), aryl, substituted aryl, substituted alkoxy, amidino, fluoroalkyl, aryloxyalkyl, fluoroalkyl, aryloxyalkyl, heterocyclic radical, substituted heterocyclic radical, and nitroalkyl; where m is from 1 to 8. Preferred non-interfering radicals are C₁-C₁₀ alkyl, C₂-C₁₀ alkenyl, C₁-C₁₀ alkoxy, C₇-C₁₂ aralkyl, C₇-C₁₂ alkaryl, C₃-C₁₀ cycloalkyl, C₃-C₁₀ cycloalkenyl, phenyl, -(CH₂)ₘ-O-(C₁-C₁₀ alkyl), aryl, and substituted aryl.

"Cₓ-C_{y} alkyl" means a straight or branched chain hydrocarbon of between x and y carbon atoms.

"Cₓ-C_{y} cycloalkyl" means a ring of between x and y carbon atoms having at least one fully saturated bond.

"Aryl" means one or more aromatic rings, each of 5 or 6 carbon atoms. Multiple aryl rings may be fused, as in naphthyl, or unfused, as in biphenyl.

"Substituted Aryl" having one or more non-interfering groups as substituents.

"Halo" means chloro, fluoro, iodo or bromo.

"Heterocycle" means one or more rings of 5, 6, or 7 atoms with or without unsaturation or aromatic character and at least one ring atom which is not carbon. Preferred heteroatoms include sulfur, oxygen, and nitrogen. Multiple rings may be fused, as in quinoline or benzofuran.

"Substituted heterocycle" means heterocycle with one or more side chains formed from non-interfering substituents.

### II. General description of the urea combinatorial library:

The urea library of the invention is a combinatorial library formed from (i) primary and/or secondary amine reactants and (ii) isocyanate reactants. Individual urea library compounds are represented by the general formula (I): 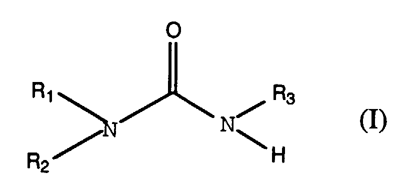 where R₁, R₂, and R₃ are substituents defined below in section V.

A preferred urea libraries is formed from diverse amine reactants and diverse isocyanate reactants.

### III. General description of the thiourea combinatorial library:

The thiourea library of the invention is a combinatorial library formed from (i) primary and/or secondary amine reactants and (ii) isothiocyanate reactants. Individual thiourea library compounds are represented by the general formula (X): 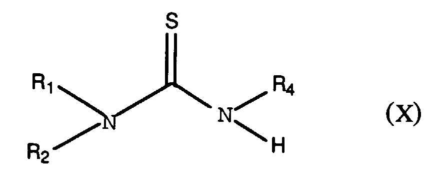 where R₁, R₂, and R₄ are substituents defined below in sections V and VII.

A preferred thiourea libraries is formed from diverse amine reactants and diverse isothiocyanate reactants.

### IV. Process parameters for making both the urea and thiourea libraries of the invention:

This invention is a general method for preparing urea or thiourea libraries. The final form of the library compounds in the urea or thiourea libraries may be as a product solute dissolved in a solvent (viz., the reaction medium) or the solvent may be removed and the final product retained as a powder, paste or oil.

The urea and thiourea library compounds of this invention are non-peptide, substantially non-naturally occurring molecules having a molecular weight range of from about 100 to about 700.

The reaction zone for forming each library compound of the urea or thiourea libraries by the method of the invention contains a solvent. The solvent reaction medium is typically a solvent not only for the library compound desired, but also for impurities such as; (i) unreacted reagent, and/or (ii) by-product(s). The impurities in the reaction mixture are generally soluble in the solution phase since they frequently have molecular weights lower than or equal to the product and share broad solubility characteristics of the product.

One method of driving a multiplicity of reactions with diverse reactants to completion is to use a stoichiometric excess of one reagent, preferably a large stoichiometric excess. For such methods the efficient removal of excess reagent may be accomplished with a solid state scavenger as taught herein. Thus, for the urea libraries, removal of excess isocyanate is the principal concern. Similarly, for thiourea libraries, the removal of excess isothiocyanate is the principal concern.

Combinatorial techniques must be very robust to work well for highly diverse groups of reactants. The solid supported scavengers employed by the processes of this invention remove excess unreacted reactant in a manner readily adapted to the diversity of reagents employed in combinatorial chemistry, particularly parallel array synthesis.

A general scheme for the use of solid phase scavengers is as follows:

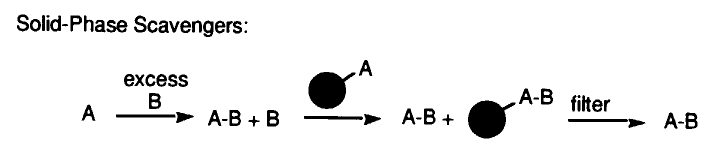

The solid supported scavenger is filtered from the liquid reaction media and the purified product A-B retained.

The utility of the method of the invention and the urea and thiourea libraries created thereby is for developing new drugs. Pharmaceutical drug discovery relies heavily on studies of structure-activity relationships wherein the structures of discovered "lead compounds" are the basis for new drug development. The method of the invention systematically and simultaneously generates large numbers of diverse urea and thiourea molecules useful as a source of lead compounds. The method of the invention systematically and simultaneously generates either a diverse library of urea and thiourea molecules useful as a source of lead compounds, or a directed library of urea and thiourea molecules useful for the optimization of lead compounds. The combinatorial urea and thiourea libraries of the invention may be screened for pharmacologically active compounds using conventional screen protocols known in the art for any targeted disease state.

### V. Process for making the diverse urea library:

The urea library of the invention is a combinatorial library formed from (i) amine reactants and (ii) isocyanate reactants. Individual urea library compounds have the general formula (I) : 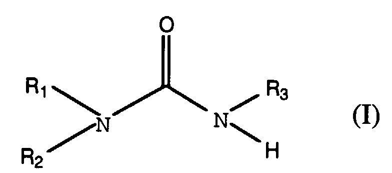 where R₁, R₂, and R₃ are substituents defined below in the sections labeled, "The amine reactant" and "The isocyanate reactant."

### The amine reactant:

Each amine reactant used in the process of making a urea combinatorial library is an amine represented by the general formula: 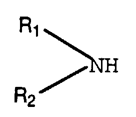 where R₁ and R₂ are independently selected from hydrogen or non-interfering substituents, with the proviso that both R₁ and R₂ may not be hydrogen. Suitable non-interfering radicals are C₁-C₁₀ alkyl, C₂-C₁₀ alkenyl, C₂-C₁₀ alkynyl, C₇-C₁₂ aralkyl, C₇-C₁₂ alkaryl, C₃-C₁₀ cycloalkyl, C₃-C₁₀ cycloalkenyl, phenyl, substituted phenyl, toluyl, xylenyl, biphenyl, C₂-C₁₂ alkoxyalkyl, C₁-C₆ alkylsulfinyl, C₁-C₁₀ alkylsulfonyl, -(CH₂)ₘ-O-(C₁-C₁₀ alkyl), aryl, substituted aryl, substituted alkoxy, amidino, fluoroalkyl, aryloxyalkyl, heterocyclic radical, substituted heterocyclic radical, and nitroalkyl; where m is from 1 to 8. Preferred non-interfering radicals are C₁-C₁₀ alkyl, C₂-C₁₀ alkenyl, C₁-C₁₀ alkoxy, C₇-C₁₂ aralkyl, C₇-C₁₂ alkaryl, C₃-C₁₀ cycloalkyl, C₃-C₁₀ cycloalkenyl, phenyl, -(CH₂)ₘ-O-(C₁-C₁₀ alkyl), aryl, and substituted aryl.

Preferably the amine reactant of step (a) is selected from the group consisting of aliphatic amines, aromatic amines, and heterocyclic amines having a molecular weight of from 50 to 600.

Examples of amine reactants suitable for use in the process of the invention are the following:
Primary Amine Reagents --
   aniline
   cyclopropylamine
   cyclobutylamine
   (-)-cis-myrtanylamine
   cyclopentylamine
   cyclohexylamine
   2-methylcyclohexylamine
   2,3-dimethylcyclohexylamine
   4-methylcyclohexylamine
   (aminomethyl)cyclohexane
   3-aminomethyl-3,5,5-trimethylcyclohexanol
   1,2,3,4-tetrahydro-1-naphthylamine
   cyclooctylamine
   l-tyrosine methyl ester
   2-(2-aminoethyl)-1-methylpyrrolidine
   n-(2-aminoethyl)pyrrolidine
   n-(3'-aminopropyl)-2-pyrrolidinone
   furfurylamine
   cyclododecylamine
   1-aminoindan
   dl-1-(1-naphthyl)ethylamine
   1-naphthalenemethylamine
   cycloheptylamine
   (1s,2s)-(+)-2-amino-1-phenyl-1,3-propanediol
   dl-2-amino-3-methyl-1-butanol
   1-isoleucinol
   1-phenylalaninol
   dl-4-chlorophenylalaninol
   d-(-)-leucinol
   l-methioninol
   histamine
   tetrahydrofurfurylamine
   dl-alpha-methyltryptamine
   tryptamine
   5-methoxytryptamine
   6-methoxytryptamine
   piperonylamine
   n-(2-aminoethyl)morpholine
   n-(3-aminopropyl)morpholine
   2-(2-aminoethylamino)-5-nitropyridine
   2-(aminomethyl)pyridine
   2-(2-aminoethyl)pyridine
   3-(aminomethyl)pyridine
   4-(aminomethyl)pyridine
   ethyl 4-amino-1-piperidinecarboxylate
   4-amino-1-benzylpiperidine
   1-(2-aminoethyl)piperidine
   1-(3-aminopropyl)-2-pipecoline
   1,2-diamino-2-methylpropane
   benzhydrylamine
   d-(-)-alpha-phenylglycinol
   1,2-diphenylethylamine
   dl-1-phenylethylamine
   (-)-norephedrine
   1,2-dimethylpropylamine
   isopropylamine
   2-methoxyisopropylamine
   dl-2-amino-1-propanol
   ethyl-3-aminobutyrate
   1,3-dimethylbutylamine
   3-amino-1-phenylbutane
   2-amino-5-diethylaminopentane
   1,5-dimethylhexylamine
   sec-butylamine
   (+/-)-2-amino-1-butanol
   3-aminopentane
   2-aminopentane
   3-aminoheptane
   2-aminoheptane
   2 -aminooctane
   benzylamine
   2-fluorobenzylamine
   2-chlorobenzylamine
   2,4-dichlorobenzylamine
   2-methoxybenzylamine
   2-ethoxybenzylamine
   2-methylbenzylamine
   3-fluorobenzylamine
   3,4-dichlorobenzylamine
   3,4-dimethoxybenzylamine
   3-(trifluoromethyl)benzylamine
   3-methylbenzylamine
   4-fluorobenzylamine
   4-chlorobenzylamine
   4-methoxybenzylamine
   4-methylbenzylamine
   2,2,2-trifluoroethylamine
   2-amino-1-phenylethanol
   1-amino-2-propanol
   3-amino-1,2-propanediol
   2,2-diphenylethylamine
   beta-methylphenethylamine
   isobutylamine
   2-methylbutylamine
   2-ethylhexylamine
   n-decylamine
   n-undecylamine
   dodecylamine
   tridecylamine
   1-tetradecylamine
   hexadecylamine
   octadecylamine
   ethylamine
   2-(2-aminoethylamino)ethanol
   2-methoxyethylamine
   2-(2-aminoethoxy)ethanol
   ethanolamine
   phenethylamine
   2-(2-chlorophenyl)ethylamine
   2-(2-methoxyphenyl)ethylamine
   3-methoxyphenethylamine
   2-(3,4-dimethoxyphenyl)ethylamine
   4-bromophenethylamine
   2-(4-chlorophenyl)ethylamine
   2-(4-methoxyphenyl)ethylamine
   tyramine
   2-(4-aminophenyl)ethylamine
   2-(p-tolyl)ethylamine
   taurine
   propargylamine
   allylamine
   3,3-dimethylbutylamine
   3,3-diphenylpropylamine
   isoamylamine
   propylamine
   3-dimethylaminopropylamine
   3-diethylaminopropylamine
   3-(di-n-butylamino)propylamine
   3-isopropoxypropylamine
   3-ethoxypropylamine
   3-amino-1-propanol
   3-phenylpropylamine
   4-amino-1-butanol
   4-phenylbutylamine
   n-amylamine
   5-amino-1-pentanol
   hexylamine
   6-amino-1-hexanol
   n-heptylamine
   n-octylamine
   n-nonylamine
   dl-2-amino-1-pentanol
   dl-2-amino-1-hexanol
   1-(3-aminopropyl)imidazole
   3,5-bis(trifluoromethyl)benzylamine
   2,4-difluorobenzylamine
   2,5-difluorobenzylamine
   2,6-difluorobenzylamine
   3,4-difluorobenzylamine
   4-(trifluoromethyl)benzylamine
   2-(trifluoromethyl)benzylamine
   4-(2-aminoethyl)benzenesulfonamide
   n-(4-aminobutyl)-n-ethylisoluminol
   n-butylamine
   2-(1-cyclohexenyl)ethylamine
   3-methoxypropylamine
   3,4,5-trimethoxybenzylamine
   3-butoxypropylamine
   aminomethylcyclopropane
   pentadecylamine
   4-(2,4-di-tert-amylphenoxy)butylamine
   3-chlorobenzylamine
   4-fluoro-alpha-methylbenzylamine
   (r)-(+)-bornylamine
   n,n-di-n-butylethylenediamine
   (r)-(-)-1-cyclohexylethylamine
   n,n,2,2-tetramethyl-1,3-propanediamine
   l-phenylalanine beta-naphthyl-amide
   2-(3-chlorophenyl)ethylamine
   2-amino-1,3-propanediol
   2-(2-thienyl)ethylamine
   2,3-dimethoxybenzylamine
   3,5-dimethoxybenzylamine
   2,4-dichlorophenethylamine
   2,5-dimethoxyphenethylamine
   3-fluoro-5-(trifluoromethyl)benzylamine
   4-(trifluoromethoxy)benzylamine
   l-leucinol
   l-leucine-4-nitroanilide
   (r)-(+)-1-(1-naphthyl)ethylamine
   (s)-(-)-1-(1-naphthyl)ethylamine
   l-valinol
   d-valinol
   d-phenylalaninol
   l-(+)-alpha-phenylglycinol
   d-(+)-alpha-methylbenzylamine
   l(-)-alpha-methylbenzylamine
   (1s,2r)-(+)-phenyl-propanolamine
   (s)-(+)-2-amino-1-propanol
   d-alaninol
   (r)-(-)-sec-butylamine
   (s)-(+)-sec-butylamine
   (s)-(+)-2-amino-1-butanol
   (r)-(-)-2-amino-1-butanol
   (r)-(-)-1-amino-2-propanol
   (s)-(+)-1-amino-2-propanol
   (s)-(-)-2-methylbutylamine
   (s)-(+)-1-cyclohexylethylamine
   oleylamine
   trimethoxysilylpropyldiethylenetriamine
   1-adamantanemethylamine
   (1s,2r)-(+)-2-amino-1,2-diphenylethanol
   (1r,2s)-(-)-2-amino-1,2-diphenylethanol
   s-benzyl-1-cysteinol
   2-(2-(aminomethyl)phenylthio)benzyl alcohol
   3-fluorophenethylamine
   2-aminobenzylamine
   2-fluorophenethylamine
   4-aminobenzylamine
   d-glucamine
   (+/-)-2,5-dihydro-2,5-dimethoxyfurfurylamine
   (s)-(+)-tetrahydrofurfurylamine
   4-fluorophenethylamine
   (1s,2s)-(+)-thiomicamine
   (-)-3,4-dihydroxynorephedrine
   (r)-(+)-1-(p-tolyl)ethylamine
   (s)-(-)-1-(p-tolyl)ethylamine
   (s)-(-)-2-amino-1,1-diphenyl-1-propanol
   (+/-)-exo-2-aminonorbornane
   (s)-(+)-2-(aminomethyl)pyrrolidine
   3-amino-1-propanol vinyl ether
   geranylamine
   4-(hexadecylamino)benzylamine
   (1r,2r,3r,5s)-(-)-isopinocampheylamine
   (1s,2s,3s,5r)-(+)-isopinocampheylamine
   n1-isopropyldiethylenetriamine
   (s)-tert-leucinol
   (r)-(-)-tetrahydrofurfurylamine
   dehydroabietylamine
   2-bromo-4,5-dimethoxyphenethylamine
   (1s,2r)-(-)-cis-1-amino-2-indanol
   (1r,2s)-(+)-cis-1-amino-2-indanol
   Additional primary amines suitable for the process of the invention are those represented by the following formulae: 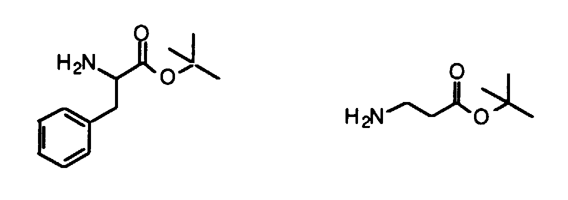 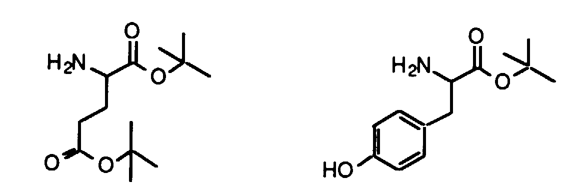 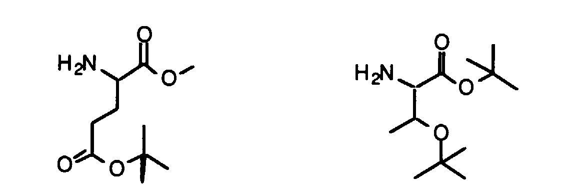 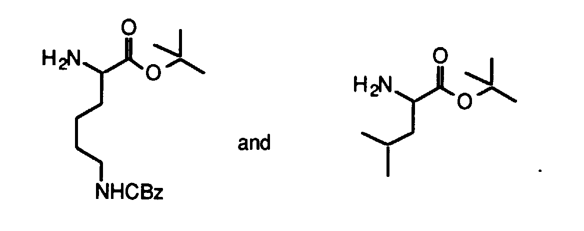
Secondary Amine Reagents --
   n-propylcyclopropanemethylamine
   (n-butylamino)acetonitrile
   n-methyl-beta-alaninenitrile
   3-(benzylamino)propionitrile
   3,3'-iminodipropionitrile
   (r)-(-)-isoproterenol
   (1r,2r)-(-)-pseudoephedrine
   l-adrenaline
   synephrine
   2-(methylamino)ethanol
   n-benzylethanolamine
   2-(ethylamino)ethanol
   diethanolamine
   2-(propylamino)ethanol
   heptamethyleneimine
   n,n',n"-methylidynetrisformamide
   n-isopropylcyclohexylamine
   n-methylcyclohexylamine
   n-ethylcyclohexylamine
   allylcyclohexylamine
   diisopropanolamine
   n-methyl-d-glucamine
   dibenzylamine
   noreleagnine
   propyleneimine
   azetidine
   n-omega-acetylhistamine
   thiazolidine
   3-pyrroline
   2,5-dimethyl-3-pyrroline
   pyrrolidine
   l-prolinamide
   l-prolinol
   3-pyrrolidinol
   n-omega-methyltryptamine
   1-piperonylpiperazine
   1,2,3,6-tetrahydropyridine
   1-phenylpiperazine
   1-(2-methoxyphenyl)piperazine
   n-(3-trifluoromethylphenyl)piperazine
   1-(4-fluorophenyl)piperazine
   1-(4-nitrophenyl)piperazine
   4-piperazinoacetophenone
   1-ethoxycarbonylpiperazine
   1-(4-chlorobenzhydryl)piperazine
   n-methylpiperazine
   1-benzylpiperazine
   1-(pyrrolidinocarbonylmethyl)piperazine
   n-isopropyl-1-piperazineacetamide
   n-beta-hydroxyethylpiperazine
   morpholine
   2,6-dimethylmorpholine
   thiomorpholine
   1,4-dioxa-8-azaspiro[4.5]decane
   piperidine
   ethyl pipecolinate
   2-methylpiperidine
   2-piperidinemethanol
   2-ethylpiperidine
   2-piperidineethanol
   n,n-diethylnipecotamide
   ethyl nipecotate
   nipecotamide
   3-methylpiperidine
   3,3-dimethylpiperidine
   3,5-dimethylpiperidine
   3-piperidinemethanol
   4-hydroxypiperidine
   4-hydroxy-4-phenylpiperidine
   4-(4-chlorophenyl)-4-hydroxypiperidine
   4-phenylpiperidine
   ethyl isonipecotate
   4-methylpiperidine
   4-benzylpiperidine
   1-(2-pyridyl)piperazine
   2-(2-methylaminoethyl)pyridine
   4-piperidinopiperidine
   1-methyl-4-(methylamino)piperidine
   decahydroquinoline
   1,2,3,4-tetrahydroisoquinoline
   hexamethyleneimine
   dimethylamine
   n-methylbenzylamine
   n-methylphenethylamine
   n'-benzyl-n,n-dimethylethylenediamine
   methylaminoacetaldehyde dimethylacetal
   n-methylpropargylamine
   dipropargylamine
   n-methylallylamine
   diallylamine
   diisopropylamine
   n-isopropylbenzylamine
   diisobutylamine
   n-methyloctadecylamine
   n-ethylmethylamine
   n-ethylbenzylamine
   diethylamine
   n,n-dimethyl-n'-ethylethylenediamine
   n,n-diethyl-n'-methylethylenediamine
   n,n,n'-triethylethylenediamine
   n-benzylglycine ethyl ester
   di-sec-butylamine
   methyl-n-propylamine
   dipropylamine
   n-methylbutylamine
   n-butylbenzylamine
   n-ethyl-n-butylamine
   dibutylamine
   di(2-ethylhexyl)amine
   dipentylamine
   di-n-hexylamine
   di-n-octylamine
   n-benzyl-2-phenylethylamine
   9-(methylaminomethyl)anthracene
   (s)-(+)-2-(methoxymethyl)pyrrolidine
   2-methylaminomethyl-1,3-dioxolane
   pindolol
   n-ethylmethallylamine
   dicyclohexylamine
   1,4,5,6-tetrahydropyrimidine
   n-(trimethylsilylmethyl)benzylamine
   4,4-dimethyl-2-imidazoline
   (s)-(+)-1-(2-pyrrolidinylmethyl)pyrrolidine
   n,n,n'-trimethylethylenediamine
   n,n,n'-trimethyl-1,3-propanediamine
   tetramethylimino-bis-propylamine
   (r)-(+)-n-benzyl-1-phenylethylamine
   n-ethylisopropylamine
   (s)-(+)-2-(anilinomethyl)pyrrolidine
   (+/-)-nornicotine
   2-(butylamino)ethanol
   4-(ethylaminomethyl)pyridine
   bis(2-methoxyethyl)amine
   4-(1-pyrrolidinyl)piperidine
   isonipecotamide
   methylisopropylamine
   n-methylhexylamine
   (r)-(+)-n-methyl-1-phenylethylamine
   3-(3-pyridylmethylamino)propionitrile
   di-n-decylamine
   piperidine thiocyanate
   1-acetylpiperazine
   n-methylhomopiperazine
   1-ethylpiperazine
   dl-adrenaline
   trans-1-cinnamylpiperazine
   (+)-pseudoephedrine
   (-)-ephedrine
   d-prolinol
   2,6-dimethylpiperidine
   (s)-(-)-n-benzyl-1-phenylethylamine
   1,3,3-trimethyl-6-azabicyclo(3.2.1)octane
   4-(4-bromophenyl)-4-piperidinol
   (s)-(-)-n-methyl-1-phenylethylamine
   n-methylhomoveratrylamine
   (r)-(+)-atenolol
   (s)-(-)-atenolol
   1-hydroxyethylethoxypiperazine
   demecolcine
   n-allylcyclopentylamine
   mitomycin c
   di-beta-d-xylopyranosylamine
   1-aza-12-crown-4
   1-(formamidomethyl)-1h-benzotriazole
   1-deoxy-1-(methylamino)-d-galactitol
   1-deoxy-1-(octylamino)-d-glucitol
   cytisine
   (r)-(+)-3-pyrrolidinol
   (+)-cis-2-benzylaminocyclohexanemethanol
   (-)-cis-2-benzylaminocyclohexanemethanol
   4-hydroxypyrimidine
   (s)-3-pyrrolidinol.
   Other suitable secondary amines for use in the process of the invention are selected from the group represented by the formula:

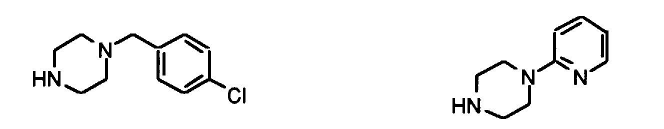

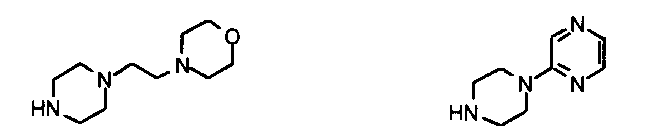

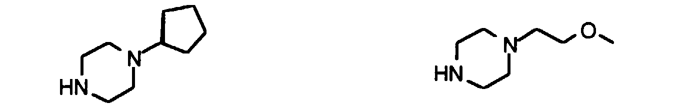

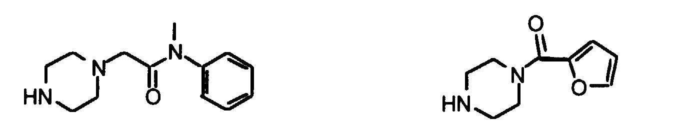

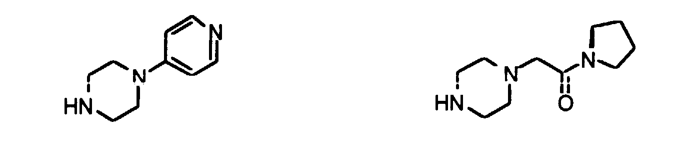

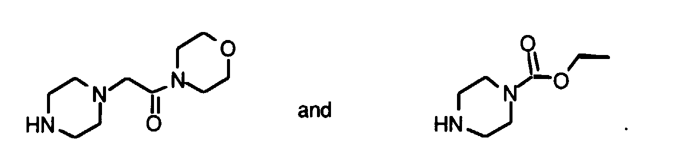

Typically, from about 8 to about 800 diverse amine reactants are employed to synthesize the urea library of the invention.

### The Isocyanate Reactant:

Each isocyanate reactant used in the process of making a urea combinatorial library is an isocyanate represented by the general formula:

R₃NCO

where R₃ is a non-interfering substituent. Suitable non-interfering radicals are C₁-C₁₀ alkyl, C₂-C₁₀ alkenyl, C₂-C₁₀ alkynyl, C₇-C₁₂ aralkyl, C₇-C₁₂ alkaryl, C₃-C₁₀ cycloalkyl, C₃-C₁₀ cycloalkenyl, phenyl, substituted phenyl, toluyl, xylenyl, biphenyl, C₂-C₁₂ alkoxyalkyl, C₁-C₆ alkylsulfinyl, C₁-C₁₀ alkylsulfonyl, -(CH₂)ₘ-O-(C₁-C₁₀ alkyl), aryl, substituted aryl, substituted alkoxy, amidino, fluoroalkyl, aryloxyalkyl, and nitroalkyl; where m is from 1 to 8. Preferred non-interfering radicals are C₁-C₁₀ alkyl, C₂-C₁₀ alkenyl, C₁-C₁₀ alkoxy, C₇-C₁₂ aralkyl, C₇-C₁₂ alkaryl, C₃-C₁₀ cycloalkyl, C₃-C₁₀ cycloalkenyl, phenyl,-(CH₂)ₘ-O-(C₁-C₁₀ alkyl), aryl, and substituted aryl.

Preferably the isocyanate reactant is selected from aromatic and aliphatic isocyanates having a molecular weight of from 50 to 600.

Examples of isocyanates suitable as reactants in the urea library making process of the invention are the following:
Isocyanate Reagents --
   trans-2-phenylcyclopropyl isocyanate
   phenyl isocyanate
   2-bromophenyl isocyanate
   2-fluorophenyl isocyanate
   2,4-difluorophenyl isocyanate
   2,6-difluorophenyl isocyanate
   2-chlorophenyl isocyanate
   2,3-dichlorophenyl isocyanate
   2,4-dichlorophenyl isocyanate
   2,5-dichlorophenyl isocyanate
   2,6-dichlorophenyl isocyanate
   2-methoxyphenyl isocyanate
   2,4-dimethoxyphenyl isocyanate
   2,5-dimethoxyphenyl isocyanate
   2-ethoxyphenyl isocyanate
   2-(trifluoromethyl)phenyl isocyanate
   o-tolyl isocyanate
   2,6-dimethylphenyl isocyanate
   2-ethylphenyl isocyanate
   3-bromophenyl isocyanate
   3-fluorophenyl isocyanate
   3-chlorophenyl isocyanate
   3,4-dichlorophenyl isocyanate
   3-methoxyphenyl isocyanate
   3-(trifluoromethyl)phenyl isocyanate
   m-tolyl isocyanate
   4-bromophenyl isocyanate
   4-fluorophenyl isocyanate
   4-chlorophenyl isocyanate
   4-methoxyphenyl isocyanate
   ethyl 4-isocyanatobenzoate
   4-(trifluoromethyl)phenyl isocyanate
   p-tolyl isocyanate
   n-(chlorocarbonyl)isocyanate
   benzoyl isocyanate
   tert-butyl isocyanate
   (s)-(-)-alpha-methylbenzyl isocyanate
   isopropyl isocyanate
   methyl isocyanate
   ethyl isocyanatoacetate
   octadecyl isocyanate
   ethyl isocyanate
   2-chloroethyl isocyanate
   allyl isocyanate
   n-propyl isocyanate
   butyl isocyanate
   cyclohexyl isocyanate
   1-naphthyl isocyanate
   (r)-(-)-1-(1-naphthyl)ethyl isocyanate
   4-fluoro-3-nitrophenyl isocyanate
   2-nitrophenyl isocyanate
   3-nitrophenyl isocyanate
   4-nitrophenyl isocyanate
   2,6-diisopropylphenyl isocyanate
   benzyl isocyanate
   3-chloropropyl isocyanate
   ethoxycarbonyl isocyanate
   3,5-bis(trifluoromethyl)phenyl isocyanate
   2,4,6-tribromophenyl isocyanate
   2,5-difluorophenyl isocyanate
   2,4,5-trichlorophenyl isocyanate
   2,4,6-trichlorophenyl isocyanate
   2-methoxycarbonylphenyl isocyanate
   2-ethoxycarbonylphenyl isocyanate
   2-isopropylphenyl isocyanate
   2,3-dimethylphenyl isocyanate
   4-methoxy-2-methylphenyl isocyanate
   2,4-dimethylphenyl isocyanate
   2,5-dimethylphenyl isocyanate
   2-ethyl-6-methylphenyl isocyanate
   3-cyanophenyl isocyanate
   5-chloro-2,4-dimethoxyphenyl isocyanate
   3-chloro-4-methylphenyl isocyanate
   3,5-dichlorophenyl isocyanate
   5-chloro-2-methoxyphenyl isocyanate
   3,4,5-trimethoxyphenyl isocyanate
   3,5-dimethoxyphenyl isocyanate
   3-(methylthio)phenyl isocyanate
   3-ethoxycarbonylphenyl isocyanate
   3-acetylphenyl isocyanate
   3,4-dimethylphenyl isocyanate
   3,5-dimethylphenyl isocyanate
   2-methoxy-5-methylphenyl isocyanate
   3-ethylphenyl isocyanate
   4-chloro-2-methoxyphenyl isocyanate
   4-chloro-2-trifluoromethylphenyl isocyanate
   4-chloro-3-trifluoromethylphenyl isocyanate
   4-iodophenyl isocyanate
   4-phenoxyphenyl isocyanate
   4-ethoxyphenyl isocyanate
   4-(methylthio)phenyl isocyanate
   4-acetylphenyl isocyanate
   4-isopropylphenyl isocyanate
   4-ethylphenyl isocyanate
   4-n-butylphenyl isocyanate
   3-(dichloromethylsilyl)propyl isocyanate
   octyl isocyanate
   4-methyl-3-nitrophenyl isocyanate
   4-chloro-2-nitrophenyl isocyanate
   2-methyl-4-nitrophenyl isocyanate
   4-methyl-2-nitrophenyl isocyanate
   2-fluoro-5-nitrophenyl isocyanate
   2-methyl-5-nitrophenyl isocyanate
   3-bromopropyl isocyanate
   2,4,6-trimethylphenyl isocyanate
   2-isopropyl-6-methylphenyl isocyanate
   2,6-diethylphenyl isocyanate
   5-chloro-2-methylphenyl isocyanate
   4-chloro-2-methylphenyl isocyanate
   4-(trifluoromethoxy)phenyl isocyanate
   4-trifluoromethylthiophenylisocyanate
   2,4-dibromophenyl isocyanate
   2,6-dibromo-4-ethylphenyl isocyanate
   2,3,4,5-tetrachlorophenyl isocyanate
   2-chloro-5-trifluoromethylphenyl isocyanate
   2-chloro-6-methylphenyl isocyanate
   2-n-carbobutoxyphenyl isocyanate
   2,4,5-trimethylphenyl isocyanate
   2-methyl-6-(t-butyl)phenyl isocyanate
   2-ethyl-6-isopropylphenyl isocyanate
   3-chloro-2-methoxyphenyl isocyanate
   3-chloro-2-methylphenyl isocyanate
   3-chloro-4-fluorophenyl isocyanate
   4-cyanophenyl isocyanate
   4-bromo-2-methylphenyl isocyanate
   4-bromo-2,6-dimethylphenyl isocyanate
   2,6-dibromo-4-fluorophenyl isocyanate
   4-n-butoxyphenyl isocyanate
   4-butoxycarbonylphenyl isocyanate
   phenethyl isocyanate
   2-methyl-3-nitrophenyl isocyanate
   hexyl isocyanate
   hexadecyl isocyanate
   methylene bis(o-chlorophenyl isocyanate)
   4-chloro-3-nitrophenyl isocyanate
   2-chloro-4-nitrophenyl isocyanate
   4,5-dimethyl-2-nitrophenyl isocyanate
   2-chloro-5-nitrophenyl isocyanate
   2-methoxy-4-nitrophenyl isocyanate
   3-fluoro-4-methylphenyl isocyanate
   5-fluoro-2-methylphenyl isocyanate
   3,5-dicarbomethoxyphenyl isocyanate
   2,4-dichlorobenzyl isocyanate
   2-(methylthio)phenyl isocyanate
   n-(methoxycarbonyl)isocyanate
   n-(phenoxycarbonyl)isocyanate n-(phenoxycarbonyl)isocyanate
   2-biphenylyl isocyanate
   3-iodophenyl isocyanate
   4-phenylphenyl isocyanate
   tetrahydro-2-pyranyl isocyanate
   4-(tert-butyl)phenylisocyanate
   1-(4-bromophenyl)ethyl isocyanate
   isocyanatoacetic acid n-butyl ester
   dodecyl isocyanate
   6,7-methylenedioxy-4-isocyanate-methylcoumarin
   (r)-(+)-alpha-methylbenzyl isocyanate
   (+/-)-1-(1-naphthyl)ethyl isocyanate
   (s)-(+)-1-(1-naphthyl)ethyl isocyanate
   3,4-difluorophenyl isocyanate
   2-methoxy-5-nitrophenyl isocyanate
   undecyl isocyanate
   ethyl 2-isocyanato-4-methyl valerate
   ethyl 6-isocyanatohexanoate
   ethyl 2-isocyanato-4-methylthiobutyrate
   ethyl 2-isocyanatopropionate
   ethyl 3-isocyanatopropionate
   ethyl 2-isocyanato-3-methylbutyrate
   tert-butyl 3-isothiocyanatopropionate
   ethyl 2-isocyanato-3-phenylpropionate
   1,3-bis(isocyanatomethyl)cyclohexane
   2-(trifluoromethoxy)phenyl isocyanate
   4-(chloromethyl) phenyl isocyanate
   1-adamantyl isocyanate
   1,3-bis(2-isocyanato-2-propyl)benzene
   n-amyl isocyanate
   n-heptyl isocyanate
   2-chloroethyl isocyanate, [ethyl-1,2-14c]
   1,1,3,3-tetramethylbutyl isocyanate
   3,5-dinitrophenyl isocyanate

### The solid supported scavenger used in making the urea libraries of the invention

The solid-supported scavenger is used in the urea library forming process of the invention. The solid-supported scavenger is represented by the formula: 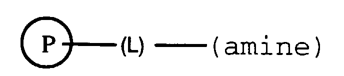 where the; 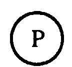 symbol is a solid-support insoluble in the liquid reaction medium used in the solution phase urea library making process. Examples of organic solid supports are polymers such as polystyrene/divinylbenzene copolymer, polyacrylamide, cellulose and polystyrene. Examples of inorganic solid supports are silica gel, alumina, and controlled pore glass.

The "(amine)" substituent of the scavenger is either a primary or secondary amine substituent, for example;

-NH₂ ,

-(CH₂)-NH₂ ,

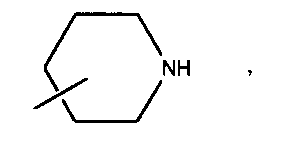

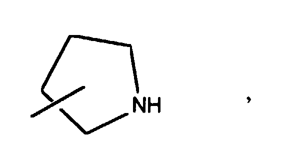

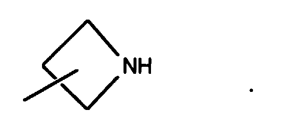

The group, -(L)-, is a linking group between the amine radical and the solid support and may be selected from a bond, or any divalent group. Useful linking groups are selected from the following:
(bond),

- O -(CH₂)ₓ -

- S -(CH₂)ₓ-

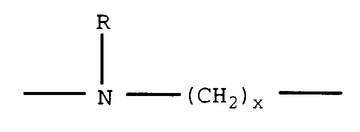

- CH₂ -(CH₂)ₓ -

where R is hydrogen or C₁-C₁₀ alkyl and x is an integer from 2 to 10.

The primary amine or secondary amine substituent on the solid supported scavenger readily reacts with excess isocyanate reagent in the urea library forming process of the invention or with excess isothiocyanate reagent in the thiourea forming process of the invention to covalently bind said excess reagents to the solid support and permit their simple removal as a solid phase. The effective available amine content of the solid supported scavenger may be readily determined by conventional chemical analysis techniques.

### VI. Detail of process steps in making the urea library:

The scavenger assisted solution phase combinatorial process for preparing urea library compounds represented by the formula; 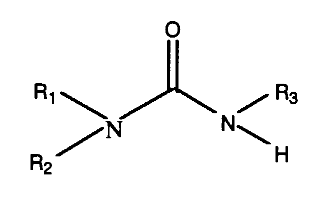 comprises the following steps:
a) adding to each reaction zone containing a liquid medium (n) equivalents of an amine reactant represented by the formula: 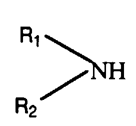 where R₁ and R₂ are independently selected from hydrogen or non-interfering substituents, with the proviso that both R₁ and R₂ are not hydrogen;
b) adding to each reaction zone of step (a) at least 1.1(n) equivalents of a solvent soluble isocyanate reactant represented by the formula,

   R₃NCO

   where R₃ is selected from a non-interfering substituent, and maintaining the reaction zone at a temperature and for a time sufficient to permit reaction of said amine and isocyanate reactants;
c) adding to the reaction zone of step (b) a solid supported amine functional scavenger represented by the formula; 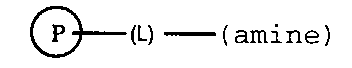 wherein; 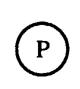 is a solid support insoluble in the liquid medium of the reaction zone, and -(L)- is a bond or a divalent linking group, (amine) is a primary or secondary amine substituent, and wherein said scavenger is added in an amount at least equal to the excess equivalents of unreacted isocyanate reactant used in step (b), and maintaining the reaction zone at a temperature and for a time sufficient to permit reaction of said excess isocyanate reactant and said scavenger;
d) separating the solid supported scavenger from the reaction mixture of step (c) and recovering in solution each substantially purified urea library compound.

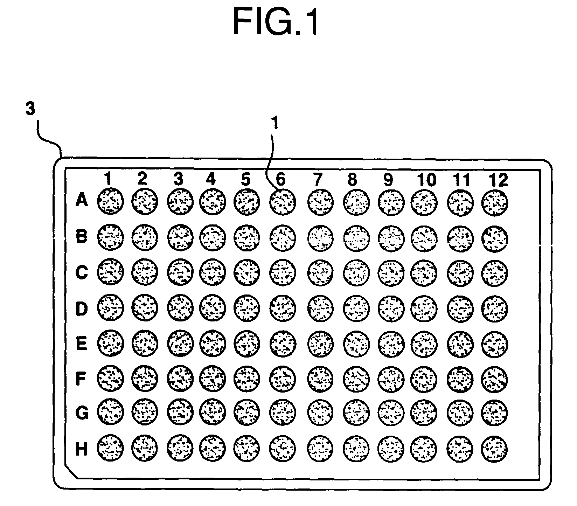

The "adding to each reaction zone" requirement for the amine and isocyanate reactants in steps (a) and (b) means that different amines and different isocyanates may be added to each reaction zone in the library forming process, if desired. In one embodiment of the process of the invention, each combination of amine and isocyanate reactants added to each library reaction zone (e.g., wells of a wellplate) is different. Thus, the same amine may be added to each row of a wellplate apparatus (as per Fig. 1) and the same isocyanate may be added to the same column of a wellplate apparatus to give a different combination of reactants in each well (viz., reaction zone) that will expectantly yield a different library compound. Alternatively, where it is desirable to have replicate samples, the same combination of amine and isocyanate reactants may be added to different reaction zones.

### Details of the Urea Library Process - Step (a):

Reaction Medium - The reaction medium may be any liquid which has the following characteristics:
(1) the amine and isocyanate reactants are capable of forming a reaction product which is substantially soluble in the reaction medium, and
(2) the solid state scavenger, both in unreacted and reacted form, is substantially insoluble in the reaction medium.

Typical reaction media useful in the processes of the invention are toluene, chloroform, methylene chloride, and acetonitrile, and tetrahydrofuran.

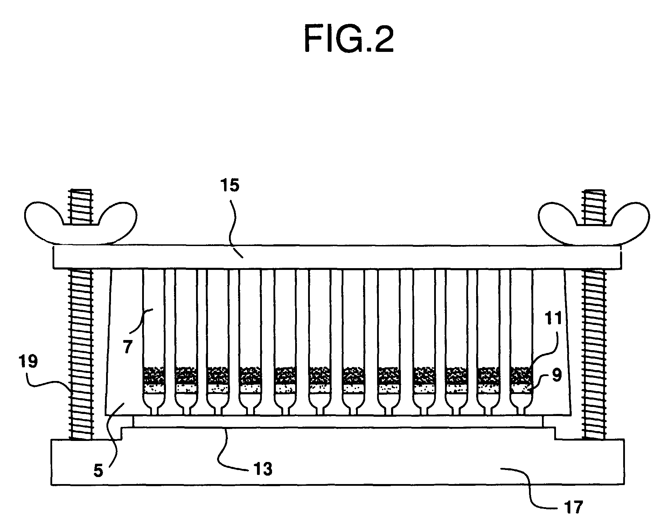

The Reaction Zone - the process of the invention may be carried out in any vessel capable of holding the liquid reaction medium and having inlet and outlet means. Preferably the process of the invention is carried out in containers adaptable to parallel array syntheses. Most preferably, the urea and thiourea library forming are done in standard wellplates, such as the 96 well wellplate illustrated in Fig. 1 and/or the wellplate apparatus illustrated in Fig. 2. Each well may be filled by multiple delivery apparatus, automated or robotic apparatus, any of which may be either manually or computer controlled.

The diverse urea library of this invention may take the form of a plurality of wellplates, each wellplate having wells containing a separate reaction product (library compound). In such cases, the library compounds are conveniently identified by their wellplate number and "x" column and "y" wellplate row coordinates.

Selection of amine reactants - A preferred technique for practicing the process of the invention is parallel array synthesis. With parallel array synthesis individual reaction products are prepared in each of multiple reaction zones. The amount of amine reactant introduced into each reaction zone will depend on the desired amount of each library compound that is needed for conducting biological assays, archival storage and other related needs. Typically, the desired amount of individual reaction product is from 1 microgram to 50 milligrams.

The amount of amine reactant in each reaction zone is represented by the symbol "(n)", where (n) represents the equivalents of amine reactant.

### Details of the Urea Library Process - Step (b) :

In the diverse urea library making process described herein the isocyanate reactant is the reactant used in excess. The method of the invention contemplates solution phase reactions where a stoichiometric excess of isocyanate reactant is used. The amount of isocyanate reactant used to insure an excess is defined as at least 1.1(n) and preferably a larger excess in the range of from 1.25(n) to 5(n), where the variable (n) is as previously defined. The stoichiometry of the reaction and calculation of equivalent weights of reagents may be done by reference to Scheme 1, infra. The 1.1 multiplier is used to insure at least a 10% stoichiometric excess of the isocyanate reactant to drive the reaction to completion, thereby removing the amine reactant from each reaction zone used to create the urea library. Thus, for example, if 1.25(n) - a 25% excess - of the isocyanate reactant is desired, then 10.7 mg. of benzylamine would be used in step (a) of the process and 18.6 mg. of 4-methoxy-phenyl isocyanate would be used in step (b) of the process.

The reaction zone is maintained at a temperature and for a time sufficient to permit reaction of the amine and isocyanate reactants, that is, to complete consumption of the amine and form an amount of urea library compound necessary to conduct biological assays to determine the efficacy of the prepared library compounds.

The time, temperature, and pressure of the combinatorial reaction zones used for the creation of library compounds are not critical aspects of the invention. Reaction times for a single step of the reaction are generally from 0.1 seconds to 24 hours, with times of 1 second to 60 minutes being most often used. The temperature of the reaction may be any temperature between the freezing point and the boiling point of the liquid reaction medium, but is generally between -10°C and +60°C, with 10°C to 40°C being preferred and ambient temperatures (about 20°C-30°C) being most preferred. The reactions may be conducted at subatmospheric pressure or superatmospheric pressure (viz., 60Kg./m² - 21000 Kg./m² absolute), but ambient atmospheric pressure (about 10330 Kg./m², absolute) is most often used.

Endpoint determination - The completion of the reaction between the amine and isocyanate reactant may be determined by a number of conventional techniques. One method is to use thin layer chromatography to determine if the amine reactant is substantially removed from the reaction zones.

Sequence of Operation - The addition of the amine and isocyanate reactants to the reaction zone may take place in any order. For example, the isocyanate reactant may be initially added to the reaction zone followed by addition of the amine reactant, or vice versa. Alternatively, the amine and isocyanate reactants may be simultaneously charged to each reaction zone.

### Details of the Urea Library Process - Step (c) :

The solid-supported scavenger addition step requires adding to the reaction mixture of step (b) a solid supported amine functional scavenger represented by the formula; 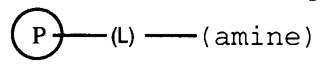 where the amount of scavenger added to the reaction product of step (b) is based on the scavenger's available amine functionality. The scavenger is added in at least an amount equal to the theoretical excess equivalents of unreacted isocyanate reactant (viz., at least 0.1 equivalents used in step (b). For example, if 0.125 mmol (18.6 mg.) of 4-methoxy phenyl isocyanate constituting a 25% (0.025 mmol) excess were used in step (b), then at least 25 mg. of an aminomethylated polystyrene having an -NH₂ content of 1 meq N/g. resin would be added in step (c). Preferably the solid supported scavenger is used in an amount that is from 1.25 to about 5 times the theoretical excess equivalents of unreacted isocyanate reactant.

The reaction zone is maintained at a temperature for a time sufficient to permit reaction of said excess isocyanate reactant and said scavenger. Typically, the reaction requires only seconds but the selection of reaction conditions that may be used is the same as set out in the preceding section (see, Details of the Urea Library Process - Step (b)).

### Details of the Urea Library Process - Step (d):

The final step in the urea library forming process of the invention is purification of the library compounds by separating the reacted and unreacted solid supported scavenger from the reaction medium of step (c) and recovering a solution of each substantially purified urea library compound.

The separation of the solid supported scavenger from the library compound dissolved in the solvent phase of the reaction may be done by any conventional chemical or physical method. Preferred are physical methods which are applicable to all members of a diverse library. Such methods include; (i) filtration, (ii) centrifugation, (iii) decantation, and (iv) washing. Filtration is a particularly preferred form of purification. It is practiced by transporting each solution of library compound through a filter medium which retains the scavenger and transfers the solution phase into a separate vessel. An apparatus using filtration is depicted in Fig. 2, infra.

The purification last step of the process may optionally be supplemented by a solvent removal step in which the solute library compound is removed from its solvent by conventional processes known in the art; such as solvent evaporation, distillation, salting out, solvent extraction, and etc.

### Examples of urea library compounds:

The following compounds are examples of selected urea library compounds prepared using the method of making a diverse urea library described above.

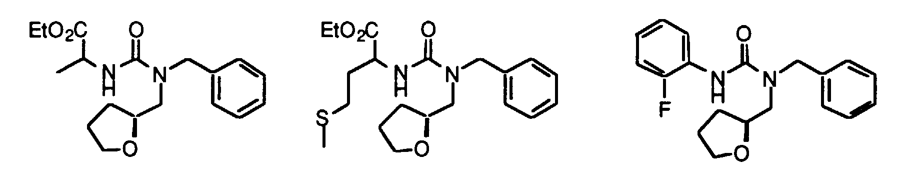

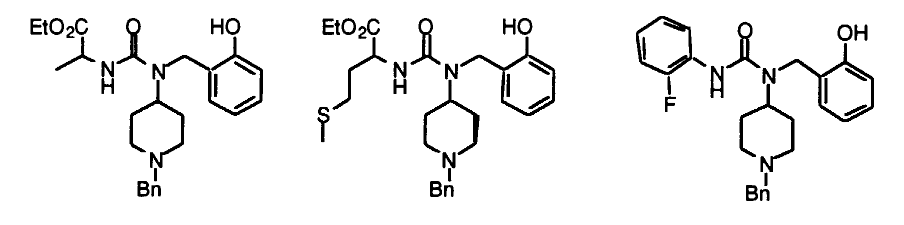

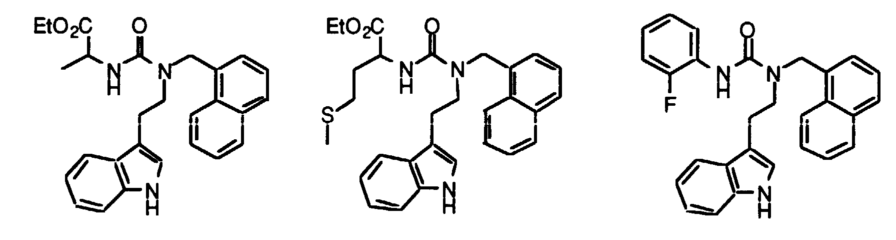

### VII. Process for making the diverse thiourea library:

The scavenger assisted solution phase combinatorial process for preparing thiourea library compounds represented by the formula (X); 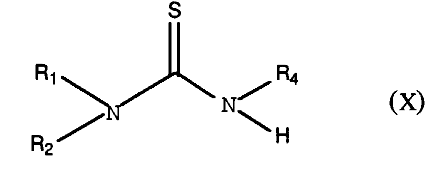 comprises the following steps:
a) adding to each reaction zone containing a liquid medium (n) equivalents of an amine reactant represented by the formula: 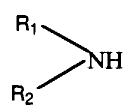 where R₁ and R₂ are independently selected from hydrogen or non-interfering substituents, with the proviso that both R₁ and R₂ may not be hydrogen;
b) adding to each reaction zone of step (a) at least 1.1(n) equivalents of a solvent soluble isothiocyanate reactant represented by the formula,

   R₄NCS

   where R₄ is selected from a non-interfering substituent, and maintaining the reaction zone at a temperature and for a time sufficient to permit reaction of said amine and isocyanate reactants;
c) adding to the reaction mixture of step (b) a solid supported amine functional scavenger represented by the formula; 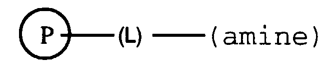 wherein; 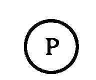 is a solid-support insoluble in the liquid medium of the reaction zone, and -(L)- is a divalent linking group, (amine) is a primary or secondary amine substituent, and wherein said scavenger is added in an amount at least equal to the excess equivalents of unreacted isothiocyanate reactant used in step (b), and maintaining the reaction zone at a temperature and for a time sufficient to permit reaction of said excess reactant and said scavenger;
d) separating the solid supported scavenger from the reaction mixture of step (c) and recovering in solution each substantially purified thiourea library compound.

The "adding to each reaction zone" requirement for the amine and isothiocyanate reactants in steps (a) and (b) means that different amines and different isothiocyanates may be added to each reaction zone in the library forming process, if desired. In one embodiment of the process of the invention, each combination of amine and isothiocyanate reactants added to each library reaction zone (e.g., wells of a wellplate) is different. Thus, the same amine may be added to each row of a wellplate apparatus (as per Fig. 1) and the same isothiocyanate may be added to the same column of a wellplate apparatus to give a different combination of reactants in each well (viz., reaction zone) that will expectantly yield a different library compound. Alternatively, where it is desirable to have replicate samples, the same combination of amine and isothiocyanate reactants may be added to different reaction zones.

### VIII. Detail of process steps in making the thiourea library:

The process for making the thiourea library of the invention is identical to the process for making the urea library of the invention as taught in the preceding sections II, III, V, and VI (which are incorporated herein by reference). Conditions for operation of the thiourea process of the invention are described by substituting the isothiocyanate reactant for every recitation of isocyanate reactant in sections II, III, V, and VI, supra. However, when isothiocyanate reactant;

R₄NCS

is substituted for the isocyanate reactant, typical reaction times and scavenging times are increased by ten to one hundred fold.

### The Isothiocyanate Reactant:

Each isothiocyanate reactant used in the process of making a thiourea combinatorial library is an isothiocyanate represented by the general formula:

R₄NCS

where R₄ is a non-interfering substituent, preferably a non-interfering substituent selected from the group; C₁-C₁₀ alkyl, C₂-C₁₀ alkenyl, C₂-C₁₀ alkynyl, C₇-C₁₂ aralkyl, C₇-C₁₂ alkaryl, C₃-C₁₀ cycloalkyl, C₃-C₁₀ cycloalkenyl, phenyl, substituted phenyl, toluyl, xylenyl, biphenyl, C₂-C₁₂ alkoxyalkyl, C₁-C₆ alkylsulfinyl, C₁-C₁₀ alkylsulfonyl,-(CH₂)ₘ-O-(C₁-C₁₀ alkyl), aryl, substituted aryl, substituted alkoxy, amidino, fluoroalkyl, aryloxyalkyl, heterocyclic radical, substituted heterocyclic radical, and nitroalkyl; where m is from 1 to 8. Preferred non-interfering radicals are C₁-C₁₀ alkyl, C₂-C₁₀ alkenyl, C₁-C₁₀ alkoxy, C₇-C₁₂ aralkyl, C₇-C₁₂ alkaryl, C₃-C₁₀ cycloalkyl, C₃-C₁₀ cycloalkenyl, phenyl, -(CH₂)ₘ-O-(C₁-C₁₀ alkyl), aryl, and substituted aryl.

Preferably the isothiocyanate reactant is selected from aromatic and aliphatic isocyanates having a molecular weight of from 73 to 600.

Examples of isothiocyanates suitable as reactants in the process of the invention are shown by the formulae below:

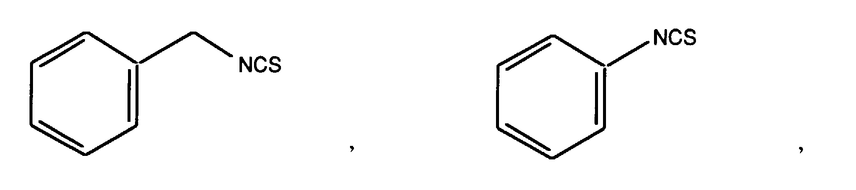

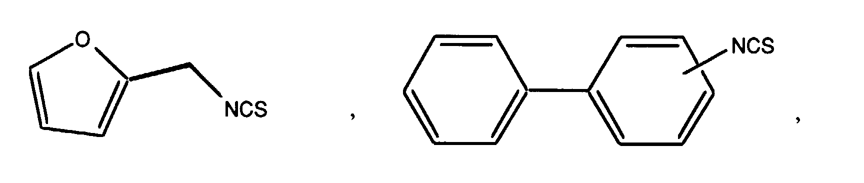

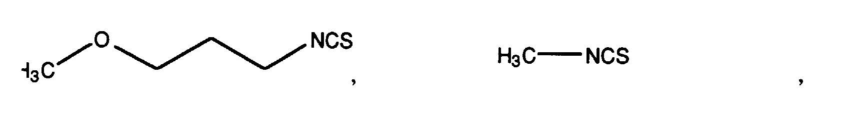

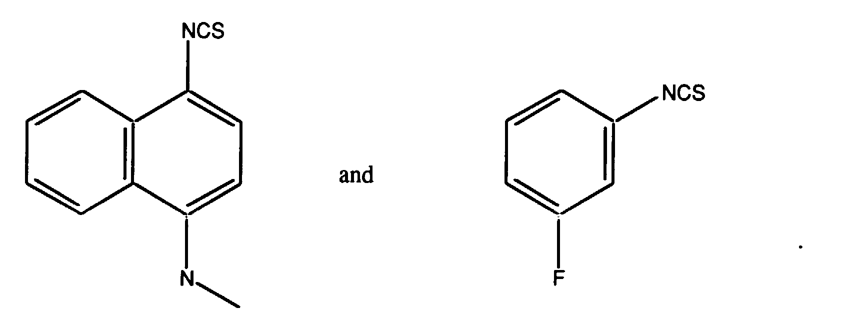

The libraries and compounds of the invention are prepared in the manner shown in the Schemes below.

Scheme 1 depicts the preferred method of making urea libraries of the invention using aminomethylated polystyrene as a solid supported scavenger.

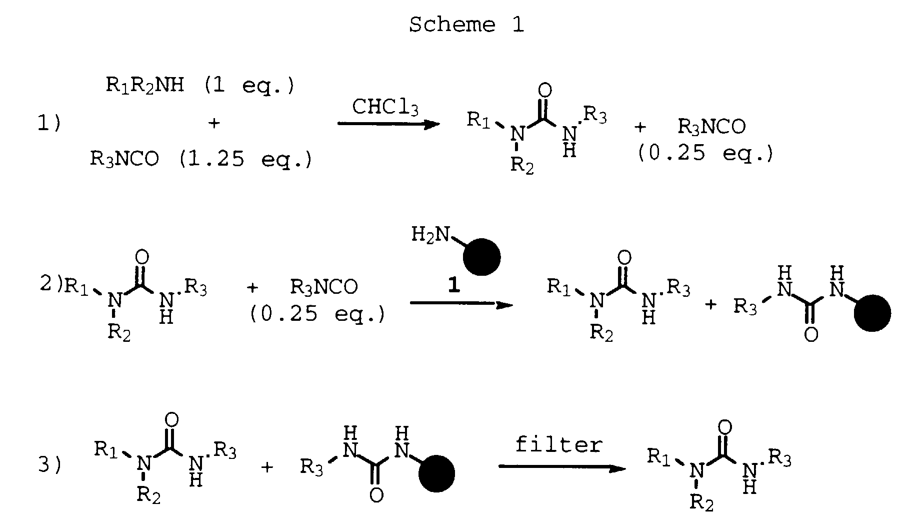

Scheme 2 depicts the preferred method of making thiourea libraries of the invention using aminomethylated polystyrene as a solid supported scavenger.

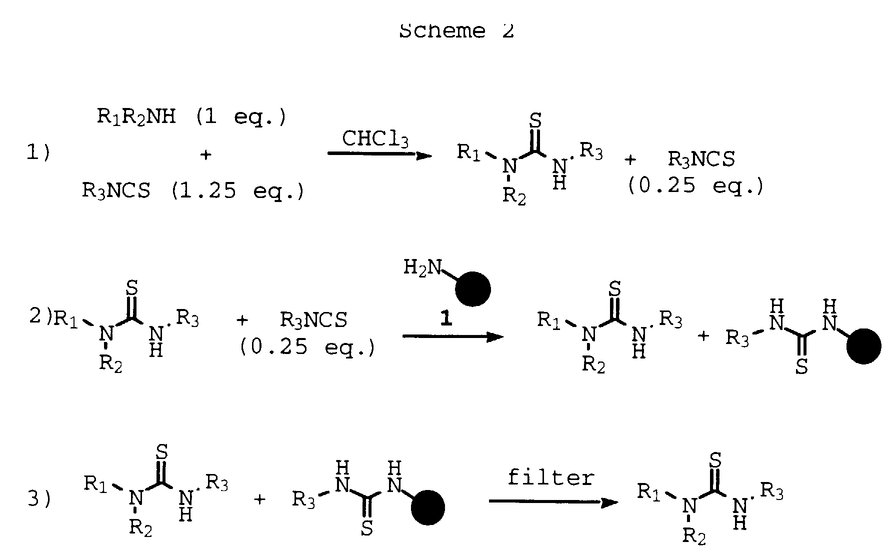

In the use of aminomethylated polystyrene **1** as a scavenger for isocyanates or isothiocyanates in the synthesis of ureas or thioureas (above), a primary or secondary amine is combined in ethanol-free chloroform with an excess of isocyanate or isothiocyanate to form a urea or thiourea as product, contaminated with starting isocyanate or isothiocyanate (Step 1). To this mixture is added an excess of aminomethylated polystyrene (source, Aldrich Chemical Co.) This resin-bound primary amine reacts to form a covalent bond between the resin-bound amine and the isocyanate or isothiocyanate, yielding a resin-bound urea or thiourea (Step 2). In the final step, the mixture of product urea or thiourea and resin in chloroform is filtered through a cotton plug and the resin, retained by the plug, is rinsed free of any product urea or thiourea by washing with additional solvent (Step 3).

### Other Utilities - Ureas:

Derivatives of ureas (capable of being prepared by the method of this invention) have utility in a number of technological areas, as follows:
A) unsaturated oligomers and their solutions are stabilized with trisubstituted ureas - US Pat. Nos. 4,672,085; 4,694,039.
B) Various alkoxyalkyl ureas are used to prepare coatings of improved stability - US Pat. No. 4,703.071.

### Other Utilities - Thioureas:

Derivatives of thioureas (capable of being prepared by the method of this invention) have utility in a number of technological areas, as follows:
A) Unsaturated oligomers and their solutions are stabilized with substituted thioureas - US Pat. No. 4,694,039.
B) Thiourea compounds are useful in immersion tin compositions for coating circuit boards - US Pat. No. 4,715,894.
C) Substituted phenyl-thioureas are useful for control of pests - US Pat. No. 5,187,197.

### The process of the invention performed in wellplate apparatus:

The process of making the urea and thiourea libraries of the invention may be conveniently carried out in a wellplate apparatus such as illustrated in Fig. 1 and Fig. 2, hereinafter described. It is particularly advantageous to carry out the method of the invention in a standard wellplate apparatus such as a plastic 96 well microtiter plate.

Typically, the wellplate apparatus is in the form of a rigid or semi-rigid plate, said plate having a common surface containing openings of a plurality of vessels arranged in rows and columns. A standard form of wellplate apparatus is a rectangular plastic plate having 8 rows and 12 columns (total 96) of liquid retaining depressions on its surface. A wellplate apparatus may optionally have other elements of structure such as a top or cover (e.g., plastic or foil), a bottom in a form such as a plate or reservoir, clamping means to secure the wellplate and prevent loss of its contained compounds.

The sequence of operations to be used for library generation with the wellplate is as follows:

### IX. The wellplate apparatus of the invention:

A wellplate inoculated with either the novel diverse urea or novel diverse thiourea compounds of the invention is itself a new construct or apparatus which has particular utility in an assay kit used to discover lead compounds. A suitable system of operation and related apparatus are made as follows:
1. Reaction zones are made by drilling 96 holes in the bottom of 96 deepwell titer plates and putting a porous frit in the bottom of each well.
2. The plate is put in a clamp assembly to seal the bottom of the wells.
3. Synthesis is begun by adding reagents (viz., the amine and isocyanate reactants) to their assigned plate coordinates (reaction zone).
4. The plate is capped then tumbled to mix the reagents.
5. Solid supported scavenger is added to each reaction zone after completion of the reaction is shown by thin layer chromatography.
6. After sufficient reaction time the plate is removed from the clamp and the resin washed.
7. The solution containing product is filtered and the solution collected by transfer into another 96 well plate.
8. The reaction products (library compounds) are analyzed by thin layer chromatography.

FIG. 1 illustrates the top surface of a wellplate apparatus of the invention. The wellplate (3) is a plastic plate with 96 wells (depressions) capable of holding liquids. When used in the parallel array synthesis individual reaction products are prepared in each well and are labeled by the wellplate coordinates. The shaded circles in the Figure represent wells filled with urea or thiourea library compounds prepared by the solution phase combinatorial process of the invention. The compound at location (1), for example, is identified by the alphanumeric coordinate, "A6."

FIG. 2 illustrates a side view of a wellplate apparatus used in the Assay Kit of the invention. The wellplate (5) contains wells (7) with a filter (9) and liquid reaction medium containing scavenger (11). The wells have an outlet at bottom which is sealed by gasket (13) held in place by top cover (15) and bottom cover (17) maintained in position by clamp (19).

### EXAMPLE

### Procedure for the Synthesis of Polymer Supported Amine Functional Scavenger

Aminomethylated polystyrene used as a scavenger may be purchased from commercial sources such as Alrdich Chemical Company or Peptides International. A literature procedure for the synthesis of aminomethylated polystyrene (from Zokos, C. C., Ferderigos, N. G. *Tetrahedron Lett.* 1995, 36, 3741-3744) is quoted below:

"Copoly(styrene-1%-divinylbenzene) resin (1 g.), N-(chloromethyl phthalimide (0.42g, 2.2 mol) and 20 ml CH₂Cl₂ were placed in a three-neck round-bottom flask equipped with an overhead stirrer. The resin was suspended by stirring and FeCl₃ (0.1g, 0.62 mmol) was added. After 2 hours of stirring, the resin was filtered and washed with dioxane, 1 N HCl:dioxane (1:1), dioxane, methanol, and dried under vacuum. The phthalimidomethyl-resin was treated under reflux with 5% hydrazine hydrate in ethanol yielding a substitution of 2 mmol N/g resin. To verify that hydrazinolysis is complete, the resins must be tested for the absence of the carbonyl group at 1720 cm⁻¹ by IR spectroscopy. Moreover, the byproduct 2,3-dihydro-1,4-phthalazinedione is insoluble in ethanol and so, washings with hot ethanol were necessary to facilitate its removal, especially from the high loading resins."

### Synthesis of a 8x11 Matrix Urea Library:

A 8x11 grid of 4 mL screw cap vials is arranged. To each of the eleven columns of vials in the grid is added 40 µmol of a differing amine (as listed below), one amine per row, as a stock solution in ethanol-free chloroform (80 mM, 500 µL). To each of the eight rows of vials in the grid is added 50 µmol each of a different isocyanate (as listed below), one isocyanate per column. Teflon lined caps are then placed on each vial and the solutions are then shaken for about one hour, at which time each vial is treated with approximately 30mg aminomethylated polystyrene (3 meq N/g, 90 mmol). The caps are replaced and the vials are shaken for an additional 24 hours, then filtered through a cotton plug and the residual resin is rinsed with three small portions of ethanol-free chloroform (approximately 200 µl). The resulting solutions are then evaporated in a fume hood under a stream of nitrogen until dry, then placed in a vacuum oven for 24 hours at room temperature. The resulting urea library compound residues are then weighed and submitted directly for testing with no further purification.

The 11 amines used in this reaction were DL-tryptophan, DL-glutamic acid, DL-tyrosine, DL-isoleucine, α-aminoisobutyric acid, 4-aminophenylacetic acid, DL-asparagine, DL-proline, trans-4-(aminomethyl)-cyclohexanecarboxylic acid, DL-phenylalanine, and 6-aminocaproic acid.

The 8 isocyanates used were 2,4-dichlorophenyl isocyanate, 4-phenylphenylisocyanate, ethyl-2-isocyanato-4-methylvalerate, ethyl-2-isocyanato-4-methylthiobutyrate, ethyl-2-isocyanatopropionate, ethyl-2-isocyanato-3-methylbutyrate, ethyl-2-isocyanato-3-phenylpropionate, and 3-ethoxycarbonylphenyl isocyanate.

### X. The Assay Kit of the Invention:

This invention is an assay kit for identification of pharmaceutical lead compounds, comprising biological assay materials and wellplate apparatus. The assay kit comprises as essential parts, (i) wellplate apparatus (containing in its wells the novel urea or thiourea library compounds of the invention), and (ii) biological assay materials.

The wellplate apparatus in the kit may comprise a set of wellplate apparatus such as illustrated in Fig. 1. The urea or thiourea library compounds contained in each wellplate are prepared by either the urea process or the thiourea library processes taught herein. Preferably the wellplate apparatus has the form of a standard 96 well microtiter plate.

The assay kit also contains biological assay materials These biological assay materials are generally in vitro tests known to be predictive of success for an associated disease state. Illustrative of biological assay materials useful in the kit of this invention are those required to conduct the following assays:
In vitro assays:
   Enzymatic Inhibition
   Receptor-ligand binding
   Protein-protein Interaction
   Protein-DNA Interaction
Cell-based, Functional assays:
   Transcriptional Regulation
   Signal Transduction/ Second Messenger
   Viral Infectivity
Add, Incubate, & Read assays:
   Scintillation Proximity Assays
      Angiotensin II SPA receptor binding assay
      Endothelin converting enzyme[¹²⁵I] SPA assay
      HIV proteinase [¹²⁵I] SPA enzyme assay
      Cholesteryl ester transfer protein (CETP) [³H] SPA assay
   Fluorescence Polarization Assays
   Fluorescence Correlation Spectroscopy
   Colorimetric Biosensors
   Ca²⁺-EGTA Dyes for Cell-based assays
   Reporter Gene Constructs for cell based assays Luciferase, green fluorescent protein, β-lactamase
   Electrical cell impedance sensor assays

## Claims

1. A scavenger assisted solution phase combinatorial process for preparing a library of compounds having a urea scaffold with three variable substituents, wherein each library compound is made in a separate reaction zone and is represented by the formula; 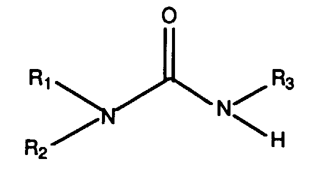 said process comprising the steps of:
a) adding to each reaction zone containing a liquid medium (n) equivalents of an amine reactant represented by the formula: 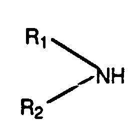 where R₁ and R₂ are independently selected from hydrogen or non-interfering substituents, with the proviso that both R₁ and R₂ may not be hydrogen;
b) adding to each reaction zone of step (a) at least 1.1(n) equivalents of a solvent soluble isocyanate reactant represented by the formula,
R₃NCO
where R₃ is selected from a non-interfering substituent, and maintaining the reaction zone at a temperature and for a time sufficient to permit reaction of said amine and isocyanate reactants;
c) adding to each reaction zone of step (b) a solid supported amine functional scavenger represented by the formula; 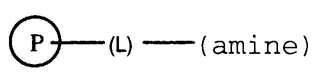 wherein; 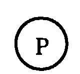 is a solid-support insoluble in the liquid reaction medium of the reaction zone, -(L)- is a divalent linking group, (amine) is either a primary or secondary amine substituent, and adding said scavenger in an amount at least equal to the excess equivalents of unreacted isocyanate reactant used in step (b), and maintaining said reaction zone at a temperature and for a time sufficient to permit reaction of said excess reactant and said scavenger;
d) separating the solid supported scavenger from each reaction zone of step (c) and recovering each substantially purified urea library compound.

2. The library of urea compounds prepared by the process of claim 1.

3. The individual urea compounds in the urea library of claim 2.

4. A scavenger assisted solution phase combinatorial process for preparing a library of compounds having a thiourea scaffold with three variable substituents, wherein each library compound is made in a separate reaction zone and is represented by the formula; 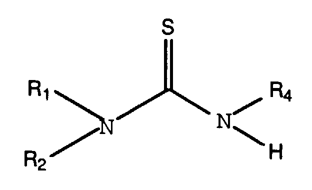 said process comprising the steps of:
a) adding to each reaction zone containing a liquid medium (n) equivalents of an amine reactant represented by the formula: 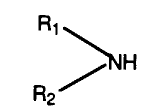 where R₁ and R₂ are independently selected from hydrogen or non-interfering substituents, with the proviso that both R₁ and R₂ may not be hydrogen;
b) adding to each reaction zone of step (a) at least 1.1(n) equivalents of a solvent soluble isothiocyanate reactant represented by the formula,
R₄NCS
where R₄ is a non-interfering substituent, and maintaining the reaction zone at a temperature and for a time sufficient to permit reaction of said amine and isothiocyanate reactants;
c) adding to each reaction zone of step (b) a solid supported amine functional scavenger represented by the formula; 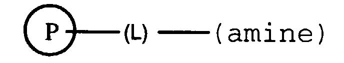 wherein; 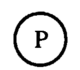 is a solid-support insoluble in the liquid medium of the reaction zone, and -(L)- is a divalent linking group, (amine) is a primary or secondary amine substituent, and adding said scavenger in an amount at least equal to the excess equivalents of unreacted isothiocyanate reactant used in step (b), and maintaining said reaction zone at a temperature and for a time sufficient to permit reaction of said excess isothiocyanate reactant and said scavenger;
d) separating the solid supported scavenger from each reaction zone of step (c) and recovering each substantially purified thiourea library compound.

5. The library of thiourea compounds prepared by the process of claim 4.

6. The individual thiourea library compounds in the thiourea library of claim 5.

7. An assay kit for identification of pharmaceutical lead compounds, comprising biological assay materials and wellplate apparatus;
wherein the improvement comprises using as wellplate apparatus a wellplate containing in each well library compound of a diverse combinatorial urea library prepared by the process of claim 1.

8. An assay kit for identification of pharmaceutical lead compounds, comprising biological assay materials and wellplate apparatus;
wherein the improvement comprises using as wellplate apparatus a wellplate containing in each well library compound of a diverse combinatorial thiourea library prepared by the process of claim 4.

9. Wellplate apparatus suitable as a replaceable element in an automated assay machine wherein the improvement comprises,
using as the wellplate apparatus a diverse urea combinatorial wellplate, wherein each well independently contains a urea library compound prepared by the method of claim 1.

10. Wellplate apparatus suitable as a replaceable element in an automated biological assay machine wherein the improvement comprises;
using as the wellplate apparatus a diverse thiourea combinatorial wellplate, wherein each well independently contains a thiourea library compound prepared by the method of claim 4.
